# EUROPEAN PATENT APPLICATION

(11) **EP 4 491 175 A1**
(43) Date of publication of application: **15.01.2025**
(21) Application number: 23386058.4
(22) Date of filing: 10.07.2023
(51) Int. Cl.: A61K 9/20, A61K 31/519

(54) **A SOLID ORAL COMPOSITION OF RUXOLITINIB**

(71) Applicant: Genepharm S.A., 15351 Pallini (GR)
(72) Inventor: Xenogiorgis, Vasileios, 17676 Athens (GR); Mpenekis, Vasilis, 16341 Athens (GR)
(74) Representative: Roukounas, Dimitrios

(57) **Abstract**

A solid oral composition comprising (R)-3-(4-(7H-pyrrolo[2,3-d]pyrimidin-4-yl)-1H-pyrazol-1-yl)-3-cyclopentylpropanenitrile or its pharmaceutically acceptable salt and one or more pharmaceutically acceptable excipients; wherein 90% or more of the (R)-3-(4-(7H-pyrrolo [2,3-d]pyrimidin-4-yl)-1H-pyrazol-1-yl)-3-cyclopentylpropanenitrile is released within 30 minutes at pH 0.1 to 6.8 at 37.0°C ± 0.5°C using a USP II paddle apparatus at stirring speed of 50 rpm.

## Description

### Technical field of the invention

The present invention relates to solid oral composition comprising a pharmaceutically active substance, (3R)-3-cyclopentyl-3-[4-(7H-pyrrolo[2,3-d] pyrimidin-4-yl) pyrazol-1-yl] propanenitrile, its pharmaceutically acceptable salts and processes for preparing the same. More specifically, the present invention relates to solid oral composition of (3R)-3-cyclopentyl-3-[4-(7H-pyrrolo[2,3-d] pyrimidin-4-yl) pyrazol-1-yl] propanenitrile, its pharmaceutically acceptable salts and processes for preparing the same, wherein 90% or more of the active ingredient is released within 30 minutes at pH 0.1 to 6.8 under ambient conditions.

### Background of the invention

(3R)-3-cyclopentyl-3-[4-(7H-pyrrolo[2,3-d] pyrimidin-4-yl) pyrazol-1-yl] propanenitrile or Ruxolitinib is reported in WO2007070514 as an inhibitor of protein kinases, such as the enzyme Janus Kinase in its subtypes 1 and 2 (hereinafter also referred to as "JAK1" and "JAK2") and is useful in the therapy of various diseases including cancer.

WO2008157208 teaches phosphate, maleate and sulfate salts of Ruxolitinib. The '208 further suggests pharmaceutical compositions of the novel salts for quick, sustained or delayed release of the active ingredient. Ruxolitinib phosphate salt described herein belongs to Biopharmaceutics Classification System class I (high permeability, high solubility) and possesses a very high aqueous solubility.

EP3183252A1 discloses oxalate salt of Ruxolitinib, its stability comparison with phosphate and pharmaceutical composition comprising the oxalate salt.

EP3183253A1 discloses besylate salt of Ruxolitinib and its stability comparison with phosphate. The specification does not disclose any specific compositions with besylate salt of Ruxolitinib.

EP3322709A1 discloses the preparation of crystalline hydrobromide salt of Ruxolitinib. The specification does not suggest any compositions with hydrobromide salt of Ruxolitinib.

EP2919766A1 teaches Sustained Release dosage forms of Ruxolitinib or a pharmaceutically acceptable salt with at least one sustained release matrix former, hydroxypropylmethylcellulose. `766 also suggests administration of the dosage form to a human results in a ratio of mean peak plasma concentration (Cₘₐₓ) to mean 12-hour plasma concentration (C₁₂ₕ) of ruxolitinib of 2 to 7, and a mean half-life (t₁₂) of from about 3.5 hours to about 11 hours. The ratio of mean peak plasma concentration (Cₘₐₓ) to mean 12-hour plasma concentration (C₁₂ₕ) & mean half-life endorse the sustained release characteristics of the inventive dosage form.

EP3218379A1 discloses Ruxolitinib salts selected from hydrobromide, hydrochloride, citrate, fumarate, tartrate, p-tosylate, benzoate, besylate, esylate, napsylate and 4-chlorobesylate. The inventive feature is in preparing the disclosed salts. There are no suggestions for compositions of the novel salts.

### Summary of the invention

The present invention provides solid oral composition comprising as active ingredient (R)-3-(4-(7H-pyrrolo[2,3-d]pyrimidin-4-yl)-1H-pyrazol-1-yl)-3-cyclopentylpropanenitrile or a pharmaceutically acceptable salt thereof and one or more pharmaceutically acceptable excipients; wherein 90% or more of the active ingredient is released from the composition within 30 minutes at pH 0.1 to 6.8 at 37.0°C ± 0.5°C using a US Pharmacopeia (USP) II paddle apparatus at stirring speed of 50 rpm.

More specifically, the present invention provides a solid oral composition comprising as active ingredient (R)-3-(4-(7H-pyrrolo[2,3-d]pyrimidin-4-yl)-1H-pyrazol-1-yl)-3-cyclopentylpropanenitrile or its pharmaceutically acceptable salt and one or more pharmaceutically acceptable excipients; wherein 90% or more of the active ingredient is released from the composition within 30 minutes at pH 0.1 to 6.8 at 37.0°C ± 0.5°C using a USP II paddle apparatus at stirring speed of 50 rpm and wherein the composition further comprises microcrystalline cellulose and lactose, or microcrystalline cellulose and lactose monohydrate, wherein the weight ratio of microcrystalline cellulose to lactose, or to lactose monohydrate, is from 0.1: 1 to 10:1.

### Detailed description of the invention

The present invention provides a solid oral composition comprising as active ingredient (R)-3-(4-(7H-pyrrolo[2,3-d]pyrimidin-4-yl)-1H-pyrazol-1-yl)-3-cyclopentylpropanenitrile or a pharmaceutically acceptable salt thereof and one or more pharmaceutically acceptable excipients. Ruxolitinib is the international nonproprietary name (INN) of (R)-3-(4-(7H-pyrrolo[2,3-d]pyrimidin-4-yl)-1H-pyrazol-1-yl)-3-cyclopentylpropanenitrile.

More specifically, the present invention provides a solid oral composition comprising as active ingredient (R)-3-(4-(7H-pyrrolo[2,3-d]pyrimidin-4-yl)-1H-pyrazol-1-yl)-3-cyclopentylpropanenitrile or a pharmaceutically acceptable salt thereof and one or more pharmaceutically acceptable excipients; wherein 90% or more of the active ingredient is released from the composition within 30 minutes at pH 0.1 to 6.8 at 37.0°C ± 0.5°C using a USP II paddle apparatus at stirring speed of 50 rpm.

The (R)-3-(4-(7H-pyrrolo[2,3-d]pyrimidin-4-yl)-1H-pyrazol-1-yl)-3-cyclopentylpropane-nitrile or its pharmaceutically acceptable salt may be present in crystalline or amorphous form.

Preferably, the D90 by volume of particles of (R)-3-(4-(7H-pyrrolo[2,3-d]pyrimidin-4-yl)-1H-pyrazol-1-yl)-3-cyclopentylpropane-nitrile or its pharmaceutically acceptable salt is from 2 microns to 100 microns, as measured by laser light diffraction, as described in European Pharmacopoeia, 11th Edition, 2.9.31.

The term "D90 by volume" means that 90% of the volume of the particles have the specified diameter.

In one embodiment of the present invention the solid oral composition comprises ^{®}-3-(4-(7H-pyrrolo[2,3-d]pyrimidin-4-yl)-1H-pyrazol-1-yl)-3-cyclopentylpropanenitrile.

In another embodiment of the present invention the solid oral composition comprises a pharmaceutically acceptable salt ^{®}(R)-3-(4-(7H-pyrrolo[2,3-d]pyrimidin-4-yl)-1H-pyrazol-1-yl)-3-cyclopentylpropanenitrile. Examples of salts which may be used in the present invention include hydrobromide, hydrochloride, citrate, fumarate, tartrate, p-tosylate, benzoate, besylate, esylate, oxalate, napsylate and 4- chlorobesylate.

Preferably, (R)-3-(4-(7H-pyrrolo[2,3-d]pyrimidin-4-yl)-1H-pyrazol-1-yl)-3-cyclopentylpropanenitrile or its pharmaceutically acceptable salt thereof is present in 0.5 weight % to 10.0 weight % of the solid oral composition.

The pharmaceutical acceptable excipients may be selected for example from fillers (also known as diluents), binders, disintegrants, lubricants, glidants, surfactants, anti-sticking agents, plasticizers coloring agents, sweeteners, flavoring agents and mixtures thereof.

Preferably, the filler is used in an amount of 60 weight % - 90 weight % of the solid oral composition. Preferably, the filler is selected from calcium phosphate, saccharose, calcium carbonate, calcium silicate, magnesium carbonate, magnesium oxide, maltodextrin, glucopyranosyl mannitol, calcium sulfate, dextrate, starch, maltose, sorbitol, lactose or lactose monohydrate, sucrose, dextrin, dextrose, hydrogenated vegetable oil, microcrystalline cellulose, or a mixture thereof. More preferably, the filler is selected from lactose, or lactose monohydrate, microcrystalline cellulose, or a mixture thereof. Even more preferably, the filler is a mixture of lactose, or lactose monohydrate, and microcrystalline cellulose. Most preferably, the filler is a mixture of microcrystalline cellulose and lactose, or microcrystalline cellulose and lactose monohydrate, wherein the weight ratio of microcrystalline cellulose to lactose, or to lactose monohydrate, ranges from 0.1: 1 to 10:1.

Preferably, the binder is used in an amount of 1 weight % to 10 weight % of the solid oral composition. Preferably, the binder is selected from microcrystalline cellulose (MCC), sodium carboxymethylcellulose, polyvinyl pyrrolidone (PVP), copovidone, polyvinyl pyrrolidone- vinyl acetate (PVP/VA) copolymer, hydroxypropylcellulose, hydroxypropyl methylcellulose, ethyl cellulose, hypromellose, or a mixture thereof.

Preferably, the disintegrant is used in an amount of 1 weight % to 15 weight % of the solid oral composition. Preferably, the disintegrant is selected from croscarmellose sodium, crospovidone, low-substituted hydroxypropylcellulose, sodium starch glycolate, or a mixture thereof.

Preferably, the lubricant is used in an amount of 0.1 weight % to 3 weight % of the solid oral composition. Preferably, the lubricant is selected from stearic acid, calcium stearate, magnesium stearate, sodium stearyl fumarate, or a mixture thereof.

Preferably, he glidant is used in an amount of 0.1 weight % to 3 weight % of the solid oral composition. Preferably, the glidant is selected from colloidal silicon dioxide, talc, or a mixture thereof.

Preferably, the sweetener is selected from the group consisting of acesulfame K, sucralose, alitame, aspartame, saccharin sodium, dipotassium glycyrrhizinate, thaumatin and the like.

Preferably, the coloring agents is selected from natural pigments and inorganic materials.

Preferably, the flavoring agent is selected from natural or synthetic materials, such as orange, spearmint, strawberry and cream flavour and the like.

The solid oral composition of the present invention may further comprise one or more surfactants. Preferably, sodium lauryl sulfate is used as surfactant, the surfactant is preferably used in an amount of 0.05 weight % to 2 weight % of the solid oral composition.

The solid oral composition of the present invention may further comprise one or more plasticizers. The plasticizer is preferably selected from dibutyl sebacetate (DBS), acetylated monoglycerides (such as the commercially available Myvacet^{®}), triacetin (also known as glycerol triacetate, or GTA), citric acid esters, like acetyltriethyl citrate (ATEC) or triethyl citrate (TEC), propylene glycol, dibutyl phthalate, diethyl phthalate, or a mixture thereof.

The solid oral composition of the present invention is preferably in the form of granules, or pellets.

The solid oral composition of the present invention remains stable after storage of 12 months at 25°C and 60% relative humidity (RH) and/or after storage of 6 months at 40°C ± 2°C and 75% RH.

The solid oral composition of the present invention may be used to prepare a dosage form, such as a unit dosage form, for example a tablet, or a capsule. Preferably, the unit dosage form is a tablet.

The total weight of the core tablet of the present invention is typically between 50 and 1000mg, preferably between 100 and 900 mg.

The tablet may be optionally film coated.

The film coating materials may be selected from hydroxypropyl cellulose, hydroxypropyl methylcellulose, ethylcellulose, povidone and copovidone, graft copolymers of polyethyleneglycol and polyvinyl alcohol (for example, the commercially available Kollicoat^{®} IR), shellac, polymers of methacrylic acid or methacrylic acid esters, methacrylic acid-methyl acrylate copolymers, polyvinyl alcohol, plasticizers such as propylene glycol, polyethylene glycol, glycerol triacetate, triethyl citrate, antitacking agents such as talc, glycerol monostearate, magnesium stearate and colorants or pigments such as titanium dioxide or iron oxide.

Coating may be effected using any conventional coating technique known in the art, such as spray coating in a conventional coating pan, or fluidized bed processor, or dip coating.

The characteristics of the tablets of the present invention may be as-follows -

### Typical Size and shape of tablets:

20 mg: oblong, biconvex tablet with dimensions 7.40 x 16.50 mm embossed with 20 on one side and plain on the other.
15 mg: oval, biconvex tablet with dimensions 7.00 x 15.00 mm embossed with 15 on one side and plain on the other.
10 mg: round, biconvex tablet with diameter 9.30 mm embossed with 10 on one side and plain on the other.
5 mg: round, biconvex tablet with diameter 7.50 mm embossed with 5 on one side and plain on the other.

### Friability

Preferably, the tablet of the present invention has friability ranging from 0.1% to 1%, more preferably from 0.1% to 0.5%, when tested by the method described in European Pharmacopoeia, 11^{th} Edition, chapter 2.9.7 (friability of uncoated tablets).

### Hardness

Preferably, the tablet of the present invention has hardness ranging from 40N to 150N, more preferably between 70N to 150N, when tested by the method described in European Pharmacopoeia, 11^{th} Edition, chapter 2.9.8 (resistance to crushing of tablets).

### Disintegration time

Preferably, the tablet of the present invention has disintegration time ranging from 0.1 minute to 5 minutes when tested by the method as described in European Pharmacopoeia, 11^{th} Edition, chapter 2.9.1.

### Dissolution studies

The solid oral composition of the present invention, preferably in the form of tablet, releases 90% or more^{®} the (R)-3-(4-(7H-pyrrolo[2,3-d]pyrimidin-4-yl)-1H-pyrazol-1-yl)-3-cyclopentylpropanenitrile within 30 minutes at pH 0.1 to 6.8 at 37.0°C ± 0.5°C using a USP II paddle apparatus at stirring speed of 50 rpm.

The dissolution method used in the present invention comprises dissolving the solid oral composition at 37.0°C ± 0.5°C in 900 ml of hydrochloric acid 0.1N having pH 1.2 and stirring it using an USP II paddle apparatus at 50 rpm.

The tablet according to the present invention is preferably packaged in primary packaging material, e.g., blisters. The tablet of the present invention is more preferably packaged in PVC/PE/PVDC blisters (triplex) or Alu-Alu blisters.

The present invention provides also a process for the preparation of a tablet^{®}mprising (R)-3-(4-(7H-pyrrolo[2,3-d]pyrimidin-4-yl)-1H-pyrazol-1-yl)-3-cyclopentylpropanenitrile. The process may be selected from wet granulation, dry granulation, direct compression and slugging. Preferably, the process is wet granulation.

Prior to preparing the solid oral composition the active ingredient could be micronized by milling, such as in an air jet mill. Preferably, the D90 by volume of the active ingre^{®}nt, i.e. of (R)-3-(4-(7H-pyrrolo[2,3-d]pyrimidin-4-yl)-1H-pyrazol-1-yl)-3-cyclopentylpropanenitrile, or a pharmaceutically acceptable salt thereof, is from 2 microns to 100 microns, as measured by laser light diffraction, as described in European Pharmacopoeia, 11th Edition, 2.9.31.

Optionally, the ingredients of the tablet of the invention are blended in order to provide a formulation having a homogenous distribution of Ruxolitinib within the composition. Blending can be carried out with conventional mixing devices, e.g., in a free-fall mixer like Turbula^{®} T10B (Bachofen AG, Switzerland). Blending can be carried out e.g., for 1 minute to 30 minutes, preferably for 2 minutes to less than 10 minutes.

The compression step can be carried out on a rotary press. If a rotary press is applied, the main compaction force usually ranges from 1 kN to 50 kN, preferably from 2 kN to 40 kN, more preferably from 3.5 kN to 30 kN. The resulting tablets usually have a hardness of 40 N to 200 N, preferably 70 N to 150 N.

### Examples

### Example 1 : Tablets comprising a composition of the present invention

### Example 1a with Ruxolitinib hydrochloride

| **Composition** | | **mg/tab** | **%** |
|---|---|---|---|
| | Ruxolitinib HCl | 22,38 | 3,50 |
| | eq. to Ruxolitinib base | 20,00 | |

| *Intragranular Excipients* | | | |
|---|---|---|---|
| | Lactose monohydrate | 140,00 | 21,88 |
| | MCC 101 | 125,00 | 19,53 |
| | Sodium Starch Glycolate | 50,00 | 7,81 |
| | Povidone K30 | 40,00 | 6,25 |
| | HydroxyPropylCellulose (Klucel ELF) | 20,00 | 3,13 |
| *Purified Water* | | *QS* | *QS* |
| *Intragranule weight* | | *397,38* | *62,10* |

| *Extragranular Excipients* | | | |
|---|---|---|---|
| | Lactose monohydrate | 110,00 | 17,19 |
| | MCC 102 | 92,62 | 14,47 |
| | Aerosil 200 | 10,00 | 1,56 |
| | Sodium Starch glycolate | 15,00 | 2,34 |
| *Pre-lubrication weight* | | *625,00* | *97,66* |

| *Lubrication* | | | |
|---|---|---|---|
| | Magnesium stearate | 15,00 | 2,34 |
| *Core Tablet weight* | | **640,00** | **100,00** |

1. The necessary quantity of PVP K30 is dissolved in purified water (PW)
2. The intragranular excipients are mixed with the API (Ruxolitinib HCl) for 5 min ± 1 min
3. The API/intragranular excipients blend of step 2 is wet granulated in a high-shear granulator
4. The wet granules are dried for the appropriate time until limit of detection (LOD) falls into the predefined limits
5. The dried granules are sized through the appropriate sieve mesh size
6. The extragranular excipients are mixed with the dried granules for 15 min ± 1 min
7. The lubricant is added in the previous blend of step 6 and mixed for 2 min ± 1 min
8. The lubricated blend is compressed into tablets

### Example 1b with Ruxolitinib hemifumarate

| **Composition** | | **mg/tab** | **%** |
|---|---|---|---|
| | Ruxolitinib Hemifumarate | 23,79 | 3,72 |
| | eq. to Ruxolitinib base | 20,00 | |

| *Intragranular Excipients* | | | |
|---|---|---|---|
| | Lactose monohydrate | 140,00 | 21,88 |
| | MCC 101 | 125,00 | 19,53 |
| | Sodium Starch Glycolate | 50,00 | 7,81 |
| | Povidone K30 | 40,00 | 6,25 |
| | HydroxyPropylCellulose (Klucel ELF) | 20,00 | 3,13 |
| *Purified Water* | | QS | QS |
| *Intragranule weight* | | *398,79* | *62,32* |

| *Extragranular Excipients* | | | |
|---|---|---|---|
| | Lactose monohydrate | 110,00 | 17,19 |
| | MCC 102 | 91,21 | 14,25 |
| | Aerosil 200 | 10,00 | 1,56 |
| | Sodium Starch glycolate | 15,00 | 2,34 |
| *Pre-lubrication weight* | | *625,00* | *97,66* |

| *Lubrication* | | | |
|---|---|---|---|
| | Magnesium stearate | 15,00 | 2,34 |
| *Core Tablet weight* | | **640,00** | **100,00** |

1. The necessary quantity of PVP K30 is dissolved in PW
2. The intragranular excipients are mixed with the API (Ruxolitinib hemifumarate) for 5 min ± 1 min
3. The API/intragranular excipients blend of step 2 is wet granulated in a high-shear granulator
4. The wet granules are dried for the appropriate time until LOD falls into the predefined limits
5. The dried granules are sized through the appropriate sieve mesh size
6. The extragranular excipients are mixed with the dried granules for 15 min ± 1 min
7. The lubricant is added in the previous blend of step 6 and mixed for 2 min ± 1 min
8. The lubricated blend is compressed into tablets

### Example 2 : Dissolution Study and physical attributes of the tablets of the present invention

The following dissolution profiles are for all 3 basic pH buffers - 0.1N HCl, pH 4.5 and pH 6.8

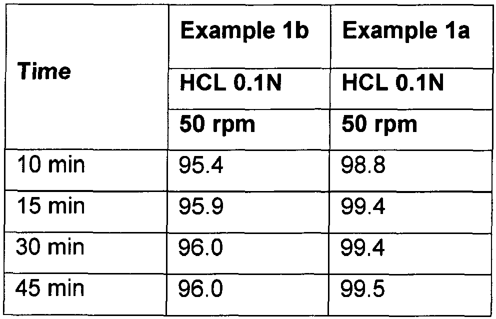

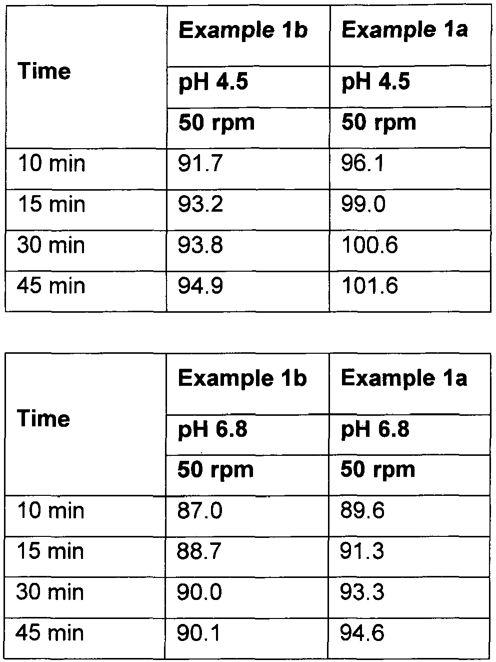

The dissolution behaviour of trials 1a and 1b found to be acceptable with a drug release of more than 85% even from the first time point (10 min) in all three pH ranges (HCl 0.1N, pH 4.5 and pH 6.8). The physical attributes of the said examples are summarized in the following table.

| **Example** | **1a** | **1b** |
|---|---|---|
| Hardness (N) | 113.5 | 97 |
| DT (min:s) | 0 min 11 s | 0 min 20 s |
| Friability (%) | 0.11 | 0.12 |
| Average weight (mg) | 639.8 | 641.2 |
| Capping | NO | NO |

## Claims

1. A solid oral composition comprising (R)-3-(4-(7H-pyrrolo[2,3-d]pyrimidin-4-yl)-1H-pyrazol-1-yl)-3-cyclopentylpropanenitrile or a pharmaceutically acceptable salt thereof and one or more pharmaceutically acceptable excipients; wherein 90% or more of (R)-3-(4-(7H-pyrrolo[2,3-d]pyrimidin-4-yl)-1H-pyrazol-1-yl)-3-cyclopentylpropanenitrile is released within 30 minutes at pH from 0.1 to 6.8 at 37.0°C ± 0.5°C using a USP II paddle apparatus at stirring speed of 50 rpm.

2. The solid oral composition as claimed in claim 1 wherein the pharmaceutically acceptable salt is selected from hydrobromide, hydrochloride, citrate, fumarate, hemifumarate, tartrate, p-tosylate, benzoate, besylate, esylate, oxalate, napsylate and 4- chlorobesylate.

3. The solid oral composition as claimed in claim 1 or 2, wherein the pharmaceutically acceptable excipient is selected from a filler, a diluent, a binder, a disintegrant, a lubricant, a glidant, a surfactant, a coloring agent, a sweetener, a flavoring agent, or a mixture thereof.

4. The solid oral composition as claimed in any one of the preceding claims, wherein the composition comprises microcrystalline cellulose (MCC), lactose, lactose monohydrate, silica, povidone, hydroxypropylcellulose, sodium starch glycolate and magnesium stearate.

5. The solid oral composition as claimed in any one of the preceding claims, wherein the composition comprises MCC and lactose, or MCC and lactose monohydrate and wherein the ratio of MCC to lactose, or to lactose monohydrate, is from 0.1 :1 to 10 :1.

6. The solid oral composition as claimed in any one of the preceding claims, wherein the amount of (R)-3-(4-(7H-pyrrolo[2,3-d]pyrimidin-4-yl)-1H-pyrazol-1-yl)-3-cyclopentylpropanenitrile or its pharmaceutically acceptable salt in the composition is from 0.5 weight % to 10.0 weight %.

7. A solid oral composition as claimed in any one of the preceding claims, wherein the D90 of (R)-3-(4-(7H-pyrrolo[2,3-d]pyrimidin-4-yl)-1H-pyrazol-1-yl)-3-cyclopentylpropanenitrile or its pharmaceutically acceptable salt is from 2 to 100 microns as measured by laser light diffraction, as described in European Pharmacopoeia, 11th Edition, 2.9.31..

8. A solid oral composition as claimed in any one of the preceding claims, wherein the composition is stable after storage of 12 months at 25°C and 60% relative humidity and/or after storage of 6 months at 40°C ± 2°C and 75% relative humidity.

9. A tablet comprising the solid oral composition as claimed in any one of the preceding claims.

10. The tablet as claimed in claim 9, wherein the tablet is film coated.

11. A solid oral composition as claimed in any one of claims 1 to 9 or a tablet as claimed in claim 10 or 11, for use in the treatment of myelofibrosis, thrombocytopenia, or graft-versus-host diseases.
